# DEMANDE DE BREVET EUROPEEN

(11) **EP 0 985 409 A1**
(43) Date de publication de la demande: **15.03.2000**
(21) Numéro de dépôt: 99402088.1
(22) Date de dépôt: 19.08.1999
(51) Int. Cl.: A61K 7/48

(54) **Utilisation en cosmétique d'acides gras**

(30) Priorité: 28.08.1998 FR 9810823
(71) Demandeur: Shiseido International France SA, 75008 Paris (FR)
(72) Inventeur: Bellon, Patrice, 75013 Paris (FR); Hornebeck, William, 02370 Chassemy (FR); Bellon, Georges, 51100 Reims (FR); Berton, Alix, 51100 Reims (FR)
(74) Mandataire: Rinuy, Santarelli

(57) **Abrégé**

Utilisation cosmétique d'un acide gras de formule (I) 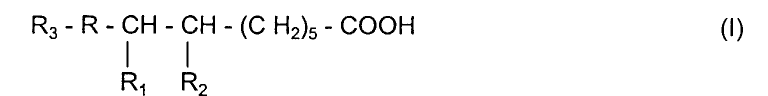 où R₁ et R₂, identiques ou différents = hydrogène ou groupe hydroxyle,
R représente un reste 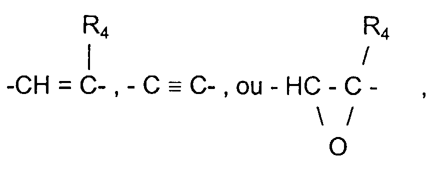 où R₄ est un hydrogène ou un groupe hydroxyle,
R₃ est un groupe aliphatique de 6 à 12 atomes de carbone et 1 à 4 insaturations indifféremment éthyléniques ou acétyléniques, l'atome de carbone en position 12 de l'acide gras de formule (I) pouvant être substitué par un groupement hydroxyle et les atomes de carbone en positions Il et 12 et/ou en positions 12 et 13 de l'acide gras pouvant former, avec un atome d'oxygène, un groupe fonctionnel époxy, ou d'un sel acceptable en cosmétologie d'un acide gras de formule (I), pour la prévention et/ou le traitement des signes du vieillissement.

## Description

La présente invention concerne l'utilisation d'acides gras insaturés ou de leurs sels en tant qu'agent anti-vieillissement ainsi que des compositions cosmétiques comprenant de tels acides gras.

On sait que le vieillissement de la peau se caractérise par des altérations des deux principaux constituants de la matrice dermique : le collagène et l'élastine. Ces altérations sont essentiellement provoquées par l'action d'enzymes protéolytiques : les métalloprotéinases matricielles (MMPs).

Il est connu que dans le cas du vieillissement chrono induit, deux métalloprotéinases matricielles sont impliquées à savoir la MMP-1 ou collagénase et la MMP-2 ou gélatinase A. Ces deux métalloprotéinases matricielles sont sécrétées par les fibroblastes du derme. Leurs teneurs dans le tissu cutané augmentent avec l'âge entraînant des altérations des fibres de collagène et d'élastine. Les signes cliniques en résultant consistent en une atrophie cutanée, une perte des propriétés mécaniques avec une perte du relief et de l'élasticité de la peau, un affaissement de la peau et une augmentation de la profondeur des rides d'expressions.

Il est par ailleurs connu que la MMP-9 ou gélatinase B et l'élastase leucocytaire sont impliquées dans le vieillissement photo induit. Ces deux enzymes sont sécrétées par les kératinocytes et/ou les polynucléaires neutrophiles au cours d'un processus inflammatoire déclenché après une exposition aux rayons U.V.. Il en résulte une dégradation et une diminution des fibres élastiques (élastose) et collagéniques. Les signes cliniques en résultant sont une hyperplasie cutanée, la formation accélérée de rides ou de stries accompagnée d'un effacement des lignes naturelles de la peau.

Ces altérations chrono ou photo induites s'accompagnent d'un remodelage tissulaire inefficace. Il s'ensuit un déséquilibre entre le catabolisme et la synthèse des macromolécules de la matrice extra-cellulaire. Il est donc nécessaire d'utiliser des substances capables de moduler l'activité des métalloprotéinases responsables de ce déséquilibre afin de prévenir les signes cliniques précédemment décrits et d'améliorer l'aspect de surface de la peau, en particulier en diminuant la profondeur des rides et en faisant disparaître les ridules.

A ce jour, la plupart des compositions cosmétiques destinées à lutter contre le vieillissement cutanée agissent par inhibition de la formation des radicaux libres, cette action étant complétée ou non par des effets antiglycation.

La formation de radicaux libres est un phénomène physiologique. Cette formation augmente de manière anormale lorsque les mécanismes internes de défense naturelle de la peau diminuent en raison, notamment, du vieillissement, ou lors de conditions extérieures particulières tel que rayonnements solaire et U.V., pollution et métaux lourds. Les radicaux libres trop nombreux altèrent les fibres de collagène et d'élastine en favorisant leur pontage et leur réticulation.

Les composés antiradicalaires, qui inhibent la formation des radicaux libres, sont le plus souvent des agents antioxydants non enzymatiques. A titre d'agent antiradicalaire on peut plus particulièrement citer les tocophérols, en particulier la vitamine E, les flavonoïdes, les composés phénoliques, les oligo-éléments comme le sélénium. Les composés antiradicalaires peuvent également consister en des antioxydants enzymatiques comme le gluthation peroxydase ou la superoxyde dismutase (SOD).

Les phénomènes de glycation apparaissent également lorsque les mécanismes internes de défense naturelle de la peau diminuent, notamment en raison du vieillissement. Ces phénomènes correspondent à une réaction non enzymatique aboutissant à la formation de liaisons entre un sucre et des protéines du collagène et de l'élastine. Les composés antiglycation sont en général des protéines qui servent de leurre à la place des lipoprotéines membranaires.

Ces composés antiradicalaires ou anti-glycation n'utilisent pas la voie enzymatique des inhibiteurs des métalloprotéinases matricielles. Par ailleurs, ils sont utilisés à des doses non adaptées, souvent trop importantes, engendrant de ce fait des effets pro-oxydant et pro-radicalaire qui aggravent le vieillissement cutané et ses signes cliniques.

Il existe donc un besoin pour des agents cosmétiques ayant une activité anti-vieillissement qui permettent d'obvier aux inconvénients des composés à action antiradicalaire et antiglycation connus.

Selon un premier objet de l'invention, celle-ci concerne l'utilisation en cosmétique de composés actifs comme agent antivieillissement, obviant aux inconvénients des composés antiradicalaires et antiglycations connus.

Selon un autre objet, l'invention concerne l'utilisation en cosmétique de composés pouvant inhiber l'activité enzymatique des métalloprotéinases matricielles, en particulier des MMP-1, 2 et 9, ainsi que de l'élastase leucocytaire, et/ou assurer un rôle protecteur des tissus cutanés vis à vis de ces mêmes enzymes.

Selon encore un autre objet de l'invention, celle-ci consiste en des compositions cosmétiques comprenant des composés utiles pour lutter contre le vieillissement de la peau.

L'invention consiste alors en l'utilisation en cosmétique d'un acide gras de formule (I) 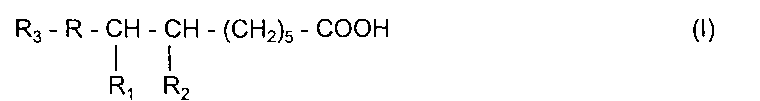 dans laquelle
R représente un resté 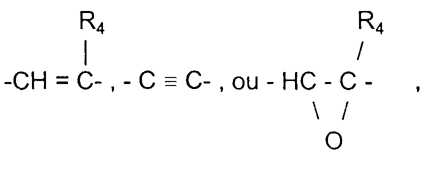 où R₄ représente un atome d'hydrogène ou un groupe hydroxyle,
R₁ et R₂, identiques ou différents, représentent un atome d'hydrogène ou un groupe hydroxyle,
R₃ représente un groupe aliphatique comprenant de 6 à 12 atomes de carbone et de 1 à 4 insaturations, ces insaturations pouvant être indifféremment éthyléniques ou acétyléniques,
l'atome de carbone en position 12 de l'acide gras de formule (I) pouvant être substitué par un groupement hydroxyle,
et les atomes de carbone en positions 11 et 12 et/ou en positions 12 et 13 dudit acide gras pouvant former, respectivement, avec un atome d'oxygène, un groupe fonctionnel époxy,
ou d'un sel acceptable en cosmétologie d'un acide gras de formule (I), pour le traitement et/ou la prévention des signes du vieillissement.

Les acides gras de formule (I) et leurs sels tels que définis ci-dessus sont plus particulièrement utiles pour lutter contre les signes du vieillissement photo- ou chrono-induit.

Selon un autre aspect de l'invention, celle-ci consiste en l'utilisation des acides gras de formule (I) et de leurs sels acceptables en cosmétologie pour inhiber l'activité enzymatique des métalloprotéinases matricielles, en particulier les MMP-1, 2 et 9 et l'élastase leucocytaire et/ou assurer un rôle protecteur des tissus cutanés vis à vis de ces mêmes enzymes.

Selon un aspect préféré de l'invention, R représente un reste -CH = CH-.

Selon un autre aspect préféré de l'invention R₁ et R₂ représentent chacun un atome d'hydrogène.

Selon encore un aspect préféré de l'invention, l'acide gras de formule (I) comprend 18 atomes de carbone.

Lorsque R représente un reste -CH = CH-, la liaison éthylénique est de préférence de conformation trans. Les autres saturations éthyléniques ou acétylénique des acides gras de formule (I) peuvent être indifféremment cis ou trans.

L'acide gras de formule (I) est préférentiellement choisi dans le groupe constitué par l'acide élaïdique de formule (Il) 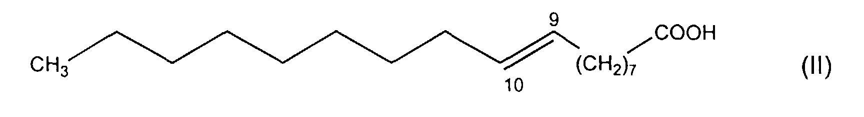 l'acide trans-parinarique ou acide octodécatétraènoïque de formule (III) 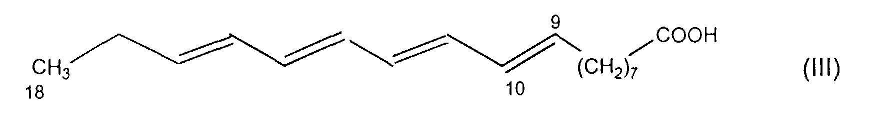 ou l'acide cis-parinarique ou acide octadécatétraénoïque de formule (IV)

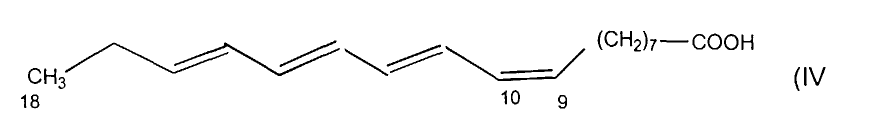

Dans le cadre de la présente invention, l'acide trans-parinarique est tout particulièrement préféré.

L'acide élaïdique est un acide gras qui peut être obtenu par synthèse chimique ou par transformation de l'acide oléique naturel ou synthétique. L'acide oléique correspond à l'isomère cis de l'acide élaïdique. Ce dernier peut être préparé en effectuant une réaction dite "d'élaïdisation", catalysée par des radiations U.V.. Cette réaction est connue de l'homme de métier.

L'acide parinarique, de forme trans ou cis, peut être préparé par synthèse chimique. Il existe également à l'état naturel dans des huiles végétales. Ainsi l'huile de Parinari, obtenue par pression des graines de plantes du genre *Parinarium* et, plus spécifiquement, du *Parinarium laurinum,* (famille des Chrysobalanacées). Les graines de ces plantes peuvent contenir environ 60 % d'acide parinarique. L'huile obtenue par pression des graines d'Impatience (*Impatiens balsamina*)*,* de la famille des Balsaminacées, peut comprendre environ 30 % d'acide parinarique. L'huile obtenue par pression des noyaux de cerise (*Prunus cesar*)*,* de la famille des Rosacées, peut contenir environ 10 % d'acide parinarique. L'acide panarique peut être extrait de ces huiles en vue de son utilisation selon l'invention. Mais ces huiles peuvent également être directement utilisées, le cas échéant, après être diluées, comme agent antivieillissement.

Les sels d'acides gras conformes à l'invention peuvent consister en des sels d'un métal alcalin, comme le sodium ou le potassium, d'ammonium, d'acides aminés, d'oligopeptides ou de peptides.

Les acides gras de formule (I) et leurs sels selon l'invention sont généralement utilisés dans des compositions cosmétiques. Ces compositions cosmétiques peuvent se présenter sous des formes galéniques adaptées à une administration topique ou orale. Toutefois, les compositions selon l'invention sont préférentiellement des compositions topiques. Ces compositions topiques sont habituellement des émulsions comprenant au moins une phase huile et au moins une phase eau. Plus particulièrement, ces émulsions peuvent être du type huile dans eau (H/E) ou eau dans huile (E/H). Elles peuvent être également des émulsions complexes du type eau dans huile dans eau (E/H/E). Elles peuvent également se présenter sous forme de microémulsions ou de nanoémulsions. Ces compositions peuvent consister en un lait, une crème, un gel ou un gel-crème, une pommade, un bâtonnet solide, une suspension ou une dispersion dans des solvants ou des corps gras, sous forme de mousse ou de spray.

Ces compositions peuvent contenir de 0,01 à 20 % en poids, de préférence de 0,5 à 10 % en poids par rapport au poids total de la composition, de l'un ou de plusieurs des acides gras décrits ci-dessus ou de leurs sels acceptables en cosmétologie. Outre les acides gras de formule (I) et leurs sels acceptables en cosmétologie, les compositions cosmétiques conformes à l'invention peuvent contenir l'un ou plusieurs des composés actifs suivants :
- des actifs anti-radicalaires, tels que des tocophérols, en particulier la vitamine E, la vitamine C, les carotènoïdes, les flavonoïdes, les composés phénoliques, des sucres ou des oligo-éléments tels que le sélénium ;
- des composés anti-glycation ;
- des composés α-hydroxy-acides ou des composés β-hydroxy-acides ;
- des actifs raffermissants ou tenseurs, tels que des protéines végétales, notamment de soja ou de blé, des extraits d'algues riches en acides aminés, ou des dérivés de silicium ;
- des actifs hydratants tels que l'acide hyaluronique, la chitine ou le chitosan, l'urée, les acides aminés, l'acide lactique et ses sels, la pyrolidone carboxylate de sodium (PCNa), la glycérine, le sorbitol et des dérivés du silicium ;
- des corps gras susceptibles de contribuer à la reconstitution du film hydrolipidique de la peau, tels que des huiles végétales riches en acides gras essentiels, en phospholipides et/ou en céramides ;
- des filtres solaires anti U.V.B et U.V.A, tels que l'octylméthoxycinnamate, le butylméthoxydibenzoyleméthane, les benzophénones ou des pigments minéraux photoprotecteurs tels que le dioxyde de titane ou l'oxyde de zinc, enrobés ou non.

En plus des composés actifs mentionnés ci-dessus, les compositions selon l'invention peuvent également comprendre des adjuvants cosmétiques classiques tels que :
- des corps gras comme des acides gras, autres que ceux de formule (I), des alcools gras, des esters d'acides gras, des huiles végétales, minérales ou de synthèse, comme des huiles siliconées volatiles ou non, des huiles fluorées ou perfluorées ;
- des solvants organiques comme les alcools monohydriques et les polyols ;
- des épaississants comme les acides polyacryliques, des gommes telles la gomme xanthane, des dérivés cellulosiques ainsi que
- des stabilisants, des émollients, des silicones, des parfums, des conservateurs, des colorants, des tensioactifs, des charges minérales ou organiques, ou des propulseurs.

De par leurs propriétés anti-vieillissement, les acides gras de formule (I) et leurs sels ainsi que les compositions qui les contiennent peuvent être utilisés pour la prévention et/ou le traitement des signes de vieillissement photo- ou chrono-induits de la peau du visage et du corps. Ils peuvent donc être utilisés comme antirides, tonifiant, lissant, raffermissant, en vue d'améliorer l'état de surface et la tonicité de la peau. Les compositions selon l'invention peuvent être utilisées alors comme compositions solaires pour le visage et le corps, dans les compositions protectrices de jour et de nuit vis à vis de la pollution ou de la fumée, sous forme de produits de soin ou de maquillage tels que fond de teint.

Les compositions selon l'invention peuvent être préparées selon des méthodes bien connues de l'homme du métier

Les acides gras de formule (I) et leurs sels selon l'invention ainsi que les compositions cosmétiques qui les contiennent, présentent des propriétés anti-vieillissement qui sont liées à une action inhibitrice des métalloprotéinases matricielles, en particulier les MMP-1, 2 et 9 et de l'élastase leucocytaire. Ils ont également une action protectrice des composants élastiques et collagéniques de la matrice dermique vis à vis de ces enzymes.

Les acides gras de formule (I) et leurs sels selon l'invention ont pour avantage de pouvoir être utilisés efficacement comme agents anti-vieillissement à des concentrations modérées. Par ailleurs ils ne sont pas prooxydants comme peuvent l'être les antioxydants utilisés comme agent anti-vieillissement selon l'art antérieur. Les acides gras selon l'invention présentent une innocuité vis à vis de la peau et, notamment, ne produisent pas d'effets irritants ou allergisants.

Les acides gras de l'invention présentent un effet inhibiteur sur les métalloprotéinases matricielles qui se traduit par un rééquilibre entre le catabolisme de la synthèse des macromolécules de la matrice extra-cellulaire (collagène et élastine). Le rééquilibrage collagène-élastine permet de réduire visiblement les signes du vieillissement.

L'acide parinarique, sous ses formes cis et trans présente plus particulièrement des propriétés inhibitrices vis à vis de la MMP-2 et de la MMP-9. L'action inhibitrice vis à vis de la MMP-2 et de la MMP-9 est plus particulièrement notable avec l'acide trans-parinarique.

On a pu noter que des peaux présentant des signes d'héliodermie traitées au moyen d'acide parinarique présentent un aspect de surface amélioré et une disparition des ridules.

De même par inhibition de la MMP-2 impliquée dans le vieillissement chrono-induit, une peau présentant des signes de vieillissement chronologique, retrouve sa tonicité et son relief.

Par ailleurs les acides gras de formule (I) et leurs sels selon la présente invention confèrent un effet protecteur en évitant l'apparition du déséquilibre entre catabolisme et synthèse des macromolécules de la matrice extracellulaire. L'équilibre permet de préserver la qualité et la quantité du collagène et de l'élastine et d'éviter l'apparition des signes du vieillissement. Plus particulièrement, il a été constaté que l'acide parinarique, sous ses forme cis et trans, de même que l'acide élaïdique, présentent des propriétés protectrices des fibres de collagène et d'élastine vis à vis de la MMP-9 et de la MMP-2. L'action protectrice vis à vis de la MMP-9 est plus forte avec l'acide élaïdique qu'avec l'acide trans-parinarique. L'action protectrice vis à vis de la MMP-2 est plus forte avec l'acide trans-parinarique qu'avec l'acide élaïdique. L'acide cis-parinarique présente en outre des propriétés protectrices notables des fibres d'élastine vis à vis de l'élastase leucocytaire.

Par effet protecteur du collagène et de l'élastine vis à vis de la MMP-9 impliquée dans le vieillissement photo-induit, l'apparition des ridules et rides lors d'expositions solaires est diminuée. De plus, par effet protecteur de l'élastine vis à vis de l'élastase leucocytaire impliquée dans le vieillissement photo-induit, l'apparition des ridules et rides lors d'expositions solaires est diminuée. Ce dernier effet protecteur a été plus particulièrement constaté par utilisation de l'acide cis-parinarique.

En outre, par effet protecteur du collagène et de l'élastine vis à vis de la MMP-2 impliquée dans le vieillissement chrono-induit, la peau conserve son état de surface, sa tonicité et son relief au cours du temps. Cet effet a été plus particulièrement noté par utilisation de l'acide trans-parinarique.

Selon un autre aspect, l'invention concerne également des compositions cosmétiques comprenant au moins un composé actif choisi dans le groupe constitué par l'acide trans-parinarique, l'acide cis-parinarique, l'acide élaïdique et leurs sels acceptables en cosmétologie. Ces compositions conviennent pour l'utilisation telle que décrite ci-dessus. Outre les composés actifs mentionnés ci-dessus, une telle composition peut comprendre d'autres actifs et des excipients conventionnels pour les compositions cosmétiques comme ceux décrits plus haut. Les compositions selon l'invention peuvent être préparées par mélange de leurs composants, en une ou plusieurs fois, éventuellement après chauffage préalable.

Les exemples qui suivent ont pour but d'illustrer la présente invention.

### Exemple 1 : Crème de jour antirides sous forme d'émulsion H/E

Cette crème est obtenue comme suit :

| Phase A | |
|---|---|
| Octyl hydroxystéarate | 5 g |
| Polypropylène glycol-15 stéaryl éther | 5 g |
| Glycéryl stéarate | 2 g |
| MYRJ 49 ² | 1,8 g |
| Acide trans-parinarique | 2 g |
| Acide élaïdique | 1 g |
| Acide cis-parinarique | 1 g |
| Parabens | qs |

| Phase B | |
|---|---|
| Eau | qs |
| Glycérine | 3 g |
| Butylène glycol | 2 g |
| Adjuvants (BHT (butyl hydroxytoluène) | qs |
| conservateurs, colorants, EDTA) | |

| Phase C | |
|---|---|
| SEPIGEL 305® ¹ | 2 g |

| Phase D | |
|---|---|
| Parfum | qs |

| | |
|---|---|
| 1. gélifiant commercialisé par la société SEPPIC | |
| 2. émulsionnant commercialisé par la société ICI | |

On chauffe la phase A à 80°C. On chauffe la phase B à 80°C. On incorpore la phase A dans la phase B et on refroidit à 45°C sous agitation. On ajoute la phase C puis la phase D.

### Exemple 2 : Crème contour des yeux antirides sous forme d'émulsion H/E

Cette crème est obtenue comme suit :

| Phase A | |
|---|---|
| MONTANOV®68 ¹ | 5 g |
| Acide trans-parinarique | 5 g |
| Isostéarate d'isostéaryle | 3 g |
| DOW CORNING 556 ² | 3 g |
| Stéaryl heptanoate et stéaryl caprylate | 3 g |
| Parabens | qs |

| Phase B | |
|---|---|
| Eau | qs |
| Gomme xanthane | 0,3 g |

| Phase C | |
|---|---|
| Adjuvants (BHT, conservateurs, colorants, EDTA) | qs |
| Protéine de soja | 2 g |
| vitamine E acétate parfum | 0,1 g |
| Parfum | qs |

| | |
|---|---|
| 1. émulsionnant commercialisé par la société SEPPIC | |
| 2. huile siliconée commercialisée par la société DOW CORNING | |

On chauffe la phase A à 80°C. On chauffe la phase B à 80°C. On incorpore la phase B dans la phase A et on refroidit à 45°C sous agitation. On ajoute la phase C.

### Exemple 3 : Lait solaire sous forme d'émulsion H/E

Ce lait est obtenu comme suit :

| Phase A | |
|---|---|
| Octyl méthoxycinnamate | 5 g |
| Diméthicone | 3 g |
| Acide cis-parinarique | 2,5 g |
| Acide élaïdique | 2,5 g |
| Huile minérale | 2 g |
| BRIJ 721 ² | 1,5 g |
| ARLATONE 983S ³ | 1,5 g |

| Phase B | |
|---|---|
| Eau | qs |
| Glycérine | 5 g |
| Butylène glycol | 4 g |
| Adjuvants (BHT, conservateurs, colorants, EDTA) | qs |

| Phase C | |
|---|---|
| SEPIGEL®305 ¹ | 5 g |

| Phase D | |
|---|---|
| Parfum | qs |

| | |
|---|---|
| 1. gélifiant commercialisé par la société SEPPIC | |
| 2. émulsionnant commercialisé par la société ICI | |
| 3. émulsionnant commercialisé par la société ICI | |

On chauffe la phase A à 80°C. On chauffe la phase B à 80°C. On incorpore la phase B dans la phase A et on refroidit à 45°C sous agitation. On ajoute la phase C puis la phase D.

### Exemple 4 : Gel-crème réparateur après soleil sous forme d'émulsion E/H (avec des silicones comme phase huileuse)

Ce gel-crème est obtenu comme suit :

| Phase A | |
|---|---|
| DOW CORNING 3225 C ¹ | 12 g |
| DOW CORNING 345 ² | 5 g |
| acide trans-parinarique | 5 g |
| Parfum | qs |

| Phase B | |
|---|---|
| Eau | qs |
| Butylène glycol | 25 g |
| Glycérine | 6 g |
| Alcool éthylique | 5 g |
| Adjuvants (BHT, conservateurs, colorants, mgCl2) | qs |

| | |
|---|---|
| 1. émulsionnant commercialisé par la société DOW CORNING | |
| 2. huile siliconée commercialisée par la société DOW CORNING | |

On mélange les ingrédients de la phase A à 80°C. On mélange les ingrédients de la phase B. On incorpore la phase B dans la phase A.

### Exemple 5 :

On a démontré l'effet d'inhibition d'acides gras selon l'invention vis à vis de différentes métalloprotéinases matricielles. A cet effet, on a déterminé l'activité enzymatique de ces MMPs en utilisant un substrat synthétique fluorescent, le (7- méthoxycoumarine-4-yl) acétyl-Pro-Leu-Gly-Leu-(3-[2,4-dinitrophényl]-L-2,3-diaminopropionyl)-Ala-ArgNH₂. Les MMPs peuvent cliver de façon spécifique la liaison amide qui lie les acides aminés Gly et Leu de ce substrat.

L'hydrolyse s'accompagne d'une augmentation sensible de la fluorescence qui peut être suivie et quantifiée à l'aide d'un spectrofluorimètre aux longueurs d'onde choisies. Suite à la rupture de la liaison Gly-Leu, le groupement dinitrophényl ne peut plus masquer la fluorescence du groupement 7-méthoxycoumarine.

On a réalisé cette hydrolyse du substrat par la MMP-2 et la MMP-9 en absence ou en présence de différentes concentrations d'acides gras selon l'invention. Les constantes d'inhibition (Ki) de l'activité enzymatique ont pu être ainsi déterminées. Les résultats obtenus figurent dans le Tableau I ci-dessous.

**Tableau I**

| **MMPs** | **Noms communs** | **Ki acide élaïdique (µM)** | **Ki acide parinarique (µM)** | |
|---|---|---|---|---|
| | | | **Cis** | **Trans** |
| MMP-1 | Collagénase intersticielle | 2,7 | nd | nd |
| MMP-2 | Gélatinase A | 4,25 | 8 | 24 |
| MMP-3 | Stromélysine-1 | 1,8 | nd | nd |
| MMP-9 | Gélatinase B | 5,8 | 14 | 1,9 |
| nd : non déterminé | | | | |

Les résultats obtenus montrent que les acides gras selon l'invention testés permettent d'inhiber efficacement les MMPs.

### Exemple 6 :

On a procédé à l'étude ex vivo de l'efficacité des acides gras selon l'invention en tant qu'agents anti-vieillissement.

### 1) Méthode

Les études ex vivo ont été réalisées sur des coupes de peau humaine issues de prépuces d'enfants âgés de 10 à 24 mois. Les coupes mises en oeuvre étaient des coupes de congélation de 8 µm d'épaisseur, sériées.

L'effet inhibiteur de l'activité enzymatique a été déterminé de la manière suivante :
les enzymes testées étaient les suivantes : MMP-2, MMP-9 ou élastase leucocytaire. L'enzyme sous sa forme activée a été incubée pendant 15 min avec différentes concentrations d'acides gras selon l'invention, puis la solution contenant l'enzyme et l'inhibiteur a été déposée sur la coupe de peau humaine et incubée 4 à 18 heures à 37°C dans une atmosphère humide.

A la fin de l'incubation, les coupes ont été colorées avec des colorants spécifiques. Le colorant de l'élastine est la + catéchine et celui des collagènes est le rouge sirius.

L'effet protecteur de la matrice extra-cellulaire a été déterminé de la façon suivante :
les lames portant les coupes de peau ont été incubées sous agitation douce pendant 4 heures dans l'éthanol pur contenant l'acide gras selon l'invention à la concentration désirée. Puis les coupes ont été rincées et séchées. L'enzyme a alors été déposée sur la coupe, la lame a été mise à incuber en atmosphère humide à 37°C pendant 4 à 18 heures. A la fin de l'incubation, les lames ont été colorées avec les colorants spécifiques de l'élastine ou des collagènes mentionnés plus haut.

Les résultats obtenus ont été déterminés selon le principe de quantification suivant:
les fibres élastiques ont été colorées de façon spécifique par la + catéchine, et ont été quantifiées grâce à un système morphométrique précis et automatisé. Les lames ont été observées sous microscope équipé d'une caméra. Les images générées ont été converties en niveaux de gris correspondant aux composants à étudier. Les plus petites particules ne correspondant pas à ces éléments ont été éliminées de l'analyse. Un programme spécifique a permis de calculer ensuite la surface relative occupée par les fibres. Avec les fibres élastiques on a pu calculer l'aire et également le volume occupé par celles-ci, puisque leur longueur est supérieure à leur largeur. L'aire occupée par les trousseaux collagéniques a pu être quantifiée selon la même procédure.

### 2) Résultats

### 2.1) Inhibition de l'activité enzymatique.

On a étudié l'activité protéolytique de la MMP-2 sur les collagènes. Lorsque la MMP-2 est ajoutée sur les coupes de peau, on a pu observer une hydrolyse des trousseaux collagéniques, ceux-ci étant moins denses et moins nombreux. De plus l'épiderme était décollé. L'hydrolyse était de 34 % environ. Lorsque l'enzyme a été pré-incubée avec l'acide trans-parinarique 1 µM, l'hydrolyse a été diminuée, la membrane basale se décollait légèrement. Avec l'acide trans-parinarique 10 µM, l'hydrolyse était nettement diminuée, les trousseaux collagéniques étaient bien structurés, la lame basale ne se décollait plus. En comparaison, la pré-incubation de la MMP-2 avec l'acide cis-parinarique 10 µM inhibait moins l'activité enzymatique.

L'hydrolyse des trousseaux collagéniques en fonction des différentes traitements a été quantifiée et les pourcentages d'inhibition déterminés dans le Tableau Il qui suit:

**Tableau II**

| Acide Gras | Trans-parinarique | | Cis-parinarique | |
|---|---|---|---|---|
| Concentration | 1µM | 10 µM | 1µM | 10 µM |
| % d'inhibition | 12 | 56 | 18 | 40 |

Dans le cas de la MMP-9, on a étudié l'hydrolyse des fibres élastiques. En effet, à l'inverse de la MMP-2, la MMP-9 possède essentiellement une activité élastinolytique in situ.

Au niveau des fibres élastiques matures du derme moyen, on a observé une hydrolyse par la MMP-9. Les fibres élastiques étaient courtes et en paquets au lieu d'être longues et bien individualisées. L'hydrolyse était de 35 % environ. L'acide trans-parinarique pré-incubé avec l'enzyme MMP-9 inhibe l'activité de celle-ci. Cette inhibition est retrouvée avec l'isomère cis de l'acide parinarique. La quantification de l'hydrolyse des fibres élastiques par la MMP-9 en fonction des différents traitements a permis de déterminer le pourcentage d'inhibition de l'activité enzymatique. Les résultats obtenus figurent dans le Tableau III suivant :

**Tableau III**

| Acide Gras | Trans-parinarique | | Cis-parinarique | |
|---|---|---|---|---|
| Concentration | 1µM | 10 µM | 1µM | 10 µM |
| % d'inhibition | 16 | 48 | 10 | 24 |

Ces expériences ont été répétées 3 à 5 fois. Les différences observées étaient statistiquement significatives.

### 2.2) Protection des fibres élastiques et collagéniques contre l'activité des MMPs et de l'élastase leucocytaire.

On a pu constaté que des coupes préalablement incubées avec de l'acide trans-parinarique et mises en contact avec l'enzyme MMP-2, présentaient une hydrolyse des trousseaux collagéniques nettement moins marquées. Les trousseaux étaient plus denses, plus épais, leurs extrémités étaient moins effilochées, le réseau apparaissait plus dense. La même constatation a pu être faite lorsque les fibres élastiques et collagéniques étaient protégées par l'acide élaïdique. La quantification des fibres a permis d'obtenir les résultats figurant dans le Tableau IV suivant :

**Tableau IV**

| Acide gras | Acide élaïdique 10 µM | Acide trans-parinarique 10 µM |
|---|---|---|
| % de protection | 32 | 61 |

Lorsque les coupes ont été préalablement incubées avec de l'acide élaïdique, l'hydrolyse des fibres élastiques matures du derme moyen par la MMP-9 était nettement diminuée. Lorsqu'on a protégé les fibres élastiques et collagéniques avec l'acide trans-parinarique, l'hydrolyse des fibres préélastiques du derme superficiel était diminuée. Les résultats obtenus figurent dans le Tableau V ci-après :

**Tableau V**

| Acide gras | Acide élaïdique 10 µM | Acide trans-parinarique 10 µM |
|---|---|---|
| % de protection | 98 | 75 |

L'élastase leucocytaire a été étudiée d'une part comme témoin de dégradation du réseau élastique in situ mais aussi pour son intervention dans la dégradation de l'élastine in vivo, notamment après exposition aux rayonnements U.V. Il a pu être ainsi constaté que la pré-incubation de la coupe avec l'acide cis-parinarique protège contre la dégradation des fibres élastiques par l'élastase. La protection était de 58 %.

## Revendications

1. Utilisation cosmétique d'un acide gras de formule (I) 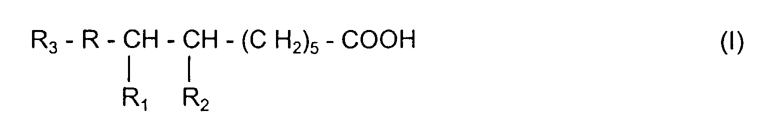 dans laquelle
R₁ et R₂, identiques ou différents représentent un atome d'hydrogène ou un groupe hydroxyle,
R représente un reste 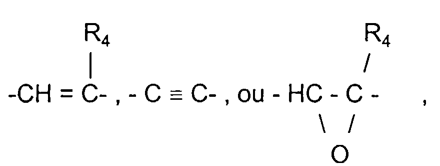 où R₄ est un atome d'hydrogène ou un groupe hydroxyle,
R₃ représente un groupe aliphatique comprenant de 6 à 12 atomes de carbone et de 1 à 4 insaturations, ces insaturations pouvant être indifféremment éthyléniques ou acétyléniques,
l'atome de carbone en position 12 de l'acide gras de formule (I) pouvant être substitué par un groupement hydroxyle et les atomes de carbone en positions 11 et 12 et/ou en positions 12 et 13 de l'acide gras pouvant former, avec un atome d'oxygène, un groupe fonctionnel époxy,
ou d'un sel acceptable en cosmétologie d'un acide gras de formule (I), pour la prévention et/ou le traitement des signes du vieillissement.

2. Utilisation selon la revendication 1 caractérisé en ce que lesdits signes du vieillissement sont chrono induits ou photo induits.

3. Utilisation d'un acide gras de formule (I) 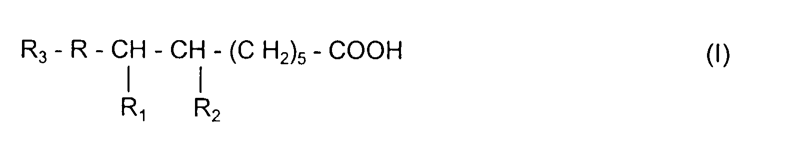 dans laquelle
R₁ et R₂, identiques ou différents représentent un atome d'hydrogène ou un groupe hydroxyle,
R représente un reste 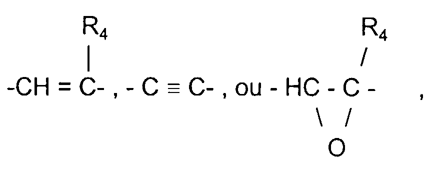 où R₄ représente un atome d'hydrogène ou un groupe hydroxyle,
R₃ représente un groupe aliphatique comprenant de 6 à 12 atomes de carbone et de 1 à 4 insaturations, ces insaturations pouvant être indifféremment éthyléniques ou acétyléniques,
l'atome de carbone en position 12 de l'acide gras de formule (I) pouvant être substitué par un groupement hydroxyle et les atomes de carbone en positions 11 et 12 et/ou en positions 12 et 13 de l'acide gras pouvant former, respectivement, avec un atome d'oxygène, un groupe fonctionnel époxy,
ou des sels acceptables en cosmétologie d'un acide gras de formule (I), pour inhiber l'activité enzymatique des métalloprotéinases matricielles et/ou assurer un rôle protecteur des tissus cutanés vis à vis des métalloprotéinases matricielles.

4. Utilisation selon la revendication 3 caractérisée en ce que les métalloprotéinases matricielles consistent en les MMP-1, MMP-2, MMP-9 et l'élastase leucocytaire.

5. Utilisation selon l'une des revendications 1 à 4, caractérisée en ce que R représente un reste -CH = CH-.

6. Utilisation selon la revendication 5 caractérisée en ce que R représente un reste -CH=CH- dont la liaison éthylénique est de conformation trans.

7. Utilisation selon l'une des revendications 1 à 6, caractérisée en ce que l'acide gras de formule (I) est choisi dans le groupe constitué par l'acide élaïdique, l'acide trans-parinarique et l'acide cis-parinarique.

8. Utilisation selon l'une des revendications 1 à 7, caractérisée en ce que l'acide gras de formule (I) ou l'un de ses sels, est contenu dans une composition cosmétique.

9. Utilisation selon la revendication 8, caractérisée en ce que ladite composition est une composition topique.
